(19) Europäisches Patentamt
European Patent Office
Office européen des brevets

(11) Publication number: **0 035 856**
**B1**

(12) # EUROPEAN PATENT SPECIFICATION

(45) Date of publication of patent specification: **04.07.84**

(21) Application number: **81300869.5**

(22) Date of filing: **03.03.81**

(51) Int. Cl.³: **A 61 K 45/06,**
A 61 K 31/44,
A 61 K 31/47,
A 61 K 31/49,
A 61 K 31/245,
A 61 K 31/585,
A 61 K 33/30,
A 61 K 31/43,
A 61 K 31/475,
A 61 K 31/485,
A 61 K 31/375

(54) **Pharmaceutical and dietary compositions.**

(30) Priority: **07.03.80 GB 8007870**

(43) Date of publication of application:
**16.09.81 Bulletin 81/37**

(45) Publication of the grant of the patent:
**04.07.84 Bulletin 84/27**

(84) Designated Contracting States:
**AT BE CH DE FR GB IT LI LU NL SE**

(56) References cited:
EP - A - 0 003 407
EP - A - 0 004 770
EP - A - 0 019 423
BE - A - 867 787
DE - A - 2 749 492
DE - A - 2 818 553
FR - A - 2 272 684
GB - A - 1 082 624

**The file contains technical information submitted after the application was filed and not included in this specification**

(73) Proprietor: **EFAMOL LIMITED**
**71/74 Mark Lane**
**London E.C.3 (GB)**

(72) Inventor: **Horrobin, David Frederick**
**P.O. Box 10 Nuns' Island**
**Montreal H3E 1J8 (CA)**

(74) Representative: **Caro, William Egerton et al,**
**J. MILLER & CO. Lincoln House 296-302 High Holborn**
**London WC1V 7JH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 035 856 B1

## Description

Field of the Invention

This invention relates to the treatment of certain diseases and disorders primarily, but not exclusively, in the field of human medicine and to compositions for use therein.

General Background

Considerable interest has been shown in recent years in the use of prostaglandin (PG) precursors in medicine.

For various reasons it is not practical to administer naturally-occurring prostaglandins such as PGE 1 and PGE 2 to patients. Consequently, considerable attention has focussed on the use of prostaglandin precursors including linoleic acid, γ-linolenic acid (GLA) and dihomo-γ-linolenic acid (DGLA).

Conversion of these materials in the body is believed to be as shown in the following diagram:

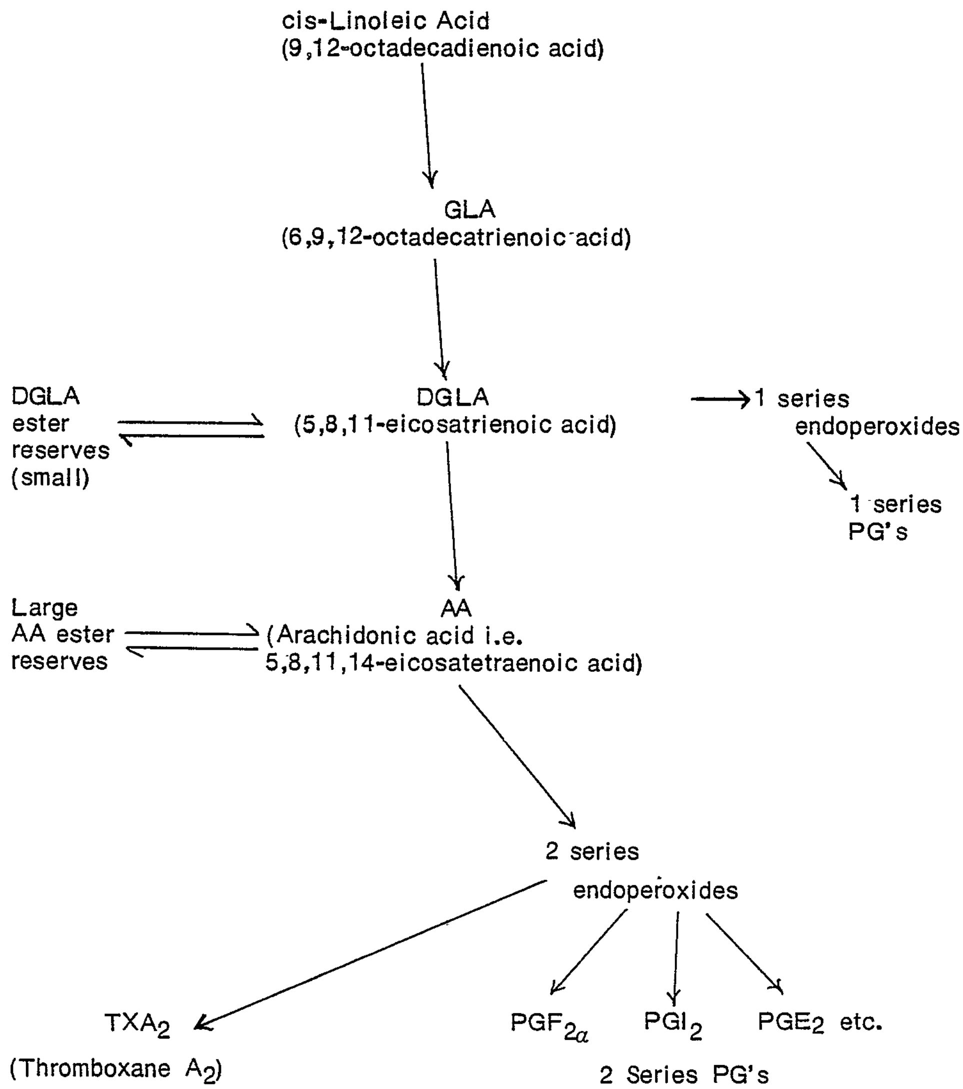


The broad outline of this pathway is well known, and it brings out clearly that a major function of essential fatty acids (EFAs) is to act as precursors for prostaglandins, 1-series PGs being formed from dihomo-γ-linolenic acid (DGLA) and 2-series PGs from arachidonic acid (AA). DGLA and AA are present in food in only small quantities, and the major EFA in food is linolenic acid which is first converted to γ-linolenic acid (GLA) and then to DGLA and AA. The conversion of linolenic acid to GLA is blocked by a high fat and high carbohydrate diet, by ageing and for example by diabetes. Stores of AA in the body in the form of lipid esters are very large indeed. In contrast only small amounts of DGLA esters are present.

Invention and Background

DGLA is the key substance. GLA is almost completely and very rapidly converted in the body to

DGLA and so for practical purposes the oral administration of DGLA and GLA amounts to the same thing. DGLA can be converted to a storage form, changed to arachidonic acid and thence to PGs of the 2-series, or converted to PGs of the 1 series.

There is increasing evidence that PGs of the 1-series play a vital role in a number of key areas. First, PGE 1 activates T lymphocytes. Defective T lymphocytes are believed to be involved in causing a wide range of allergic and inflammatory disorders and in making individuals susceptible to cancer and infections of all types. Second, PGE 1 is important in preventing over-production of collagen and fibrous tissue, a factor which plays a major role in arthritis and the so-called collagen diseases. Third, PGE 1 levels are extremely low in patients with schizophrenia and are moderately low in patients with depression. Fourth PGE 1 appears to be important in controlling cholesterol levels and necessary for the normal actions of insulin. Fifth, PGE 1 dilates blood vessels and may be expected to be helpful in any situation in which vessel spasm occurs. Sixth, PGE 1 appears to inhibit the production of 2-series PGs, levels of which are raised in a wide variety of inflammatory disorders. Seventh, PGE 1 increases a production of cyclic AMP which has anti-inflammatory effects.

There are therefore very strong reasons, and this is the broad feature of the present invention, for influencing the 1-series/2-series PG balance in the body in favour of 1-series PGs and specifically to selectively enhancing formation of PGs of the 1-series and particularly PGE 1. The diseases and disorders below are among those in which such action is indicated:

1. Situations in which defective T lymphocyte function has been described such as allergic and inflammatory disorders, multiple sclerosis, schizophrenia and cancer.

2. Situations in which regulation of collagen formation and breakdown is defective including rheumatoid arthritis, systemic lupus erythematosus, Crohn's disease and the various "collagen" diseases.

3. Mental illnesses in which low PGE 1 levels have been reported, including depression and schizophrenia. In depression, platelet PGE 1 production is moderately reduced whereas in schizophrenia it is severely reduced.

4. Disorders of lipid and carbohydrate metabolism in particular diabetes mellitus and situations in which blood cholesterol levels are elevated.

5. Disorders in which there is a tendency of blood vessels to go into spasm such as angina pectoris, myocardial infarction and Reynaud's syndrome.

Selective enhancement of 1-series PG production has been explored in human platelets. The method is given in detail later herein but briefly human platelets are incubated with radioactive DGLA or arachidonic acid. The PGs produced during incubation are extracted by conventional means and separated by thin layer chromatography, and the amount of radioactivity appearing in each PG or related substance is counted. PGE 1, PGF $1\alpha$ and thromboxane B1 from DGLA, and PGE 2, PFG $2\alpha$ and thromboxane B2 from AA are estimated. The results, as given herein, demonstrate the inventor's belief that the effects of various agents on AA and DGLA conversion can be quite different and that it is possible to selectively enhance formation of PGE 1 and other 1-series PG compounds. The effect is believed to be by influencing the conversion of DGLA to the 1-series PGs.

The balance between 1-series and 2-series PGs is, the inventor believes, significant in terms of overall control of the conversion pathways given earlier. Such control is not understood in detail but without restriction to the theory it appears first that PGE 2 is able to enhance the formation of 1-series PGs, and second that PGE 1 is able to block arachidonic acid mobilisation from tissue stores. Thus the conditions for a negative feedback control loop exist; overproduction of PGE 2 from AA will activate PGE 1 synthesis, the PGE 1 will inhibit AA mobilisation, and production of 2-series PGs will drop. Further, TXA 2, an unstable product of the 2-series endoperoxides arising in 2-series PG production, also appears to enhance 1-series PG and in particular PGE 1 production. Thus again the activity of the 2-series PG synthesis pathway gives rise indirectly to a material that controls that pathway.

Effective Agents

Thus the combination of DGLA or GLA or their esters from any natural or synthetic source with one or more of the agents discussed below is proposed for use in any condition calling for selective enhancement of 1-series PG production or more broadly for influence of the 1-series/2-series PG balance in the body in favour of 1-series PGs. Particular diseases and disorders have been listed earlier herein.

The agents are as follows, the 'action' referred to being in the 1-series/2-series PG balance as above.

1. Chloroquine and other 4-aminoquinolines having the said action on PG metabolism. Other compounds of this class in clinical use are amodiaquine and hydroxychloroquine.

2. Diidohydroxyquin and other 8-hydroxy and 8-aminoquinolines having the said action in PG metabolism. Other compounds of this class in clinical use include iodochlorhydroxyquin, chiniofon, pentaquine, isopentaquine and primaquine.

3. Quinacrine and other acridines having the said action on PG metabolism (quinacrine is also known as mepacrine). Other acridines are not in clinical use but a wide number are known.

4. Quinidine, quinine and procaine.

5. Emetine and metronidazole.

STRUCTURES

The quinoline nucleus is:

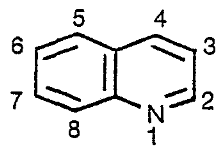

and the acridine nucleus:

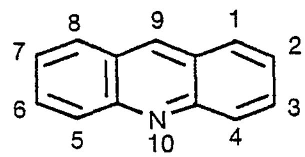

Of the 4-amino quinolines, chloroquine, which is available as chloroquine phosphate and chloroquine hydrochloride, has a chlorine at the 7-position of the quinidine nucleus and the 4-amino group substituted by:

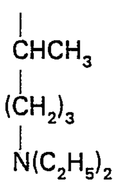

in the related amodiaquine the 4-amino group is substituted by:

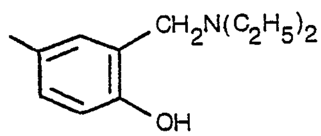

and in hydroxychloroquine by

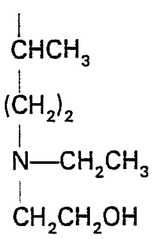

both having the 7-chloro substituent.

The structures of diiodhydroxyquin, iodochlorhydroxyquin and chiniofen are

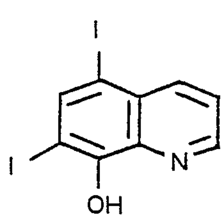

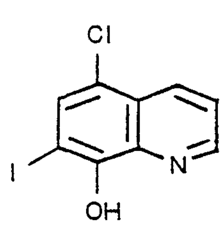

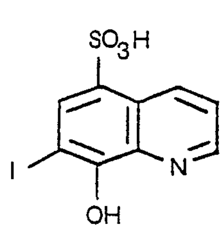

Pentaquine, isopentaquine and primaquine are 6-methoxy, 8-amino quinolines with the amino group respectively substituted by:

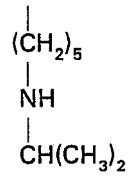

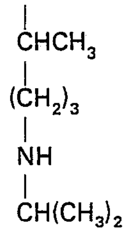

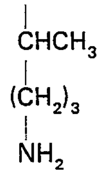

The structures of quinine and quinidine are:

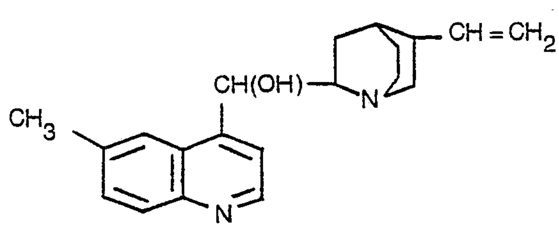

the difference being

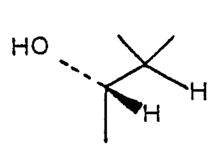

(quinidine)

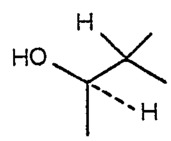

(quinine)

The structure of quinacrine (as the hydrochloride) is:

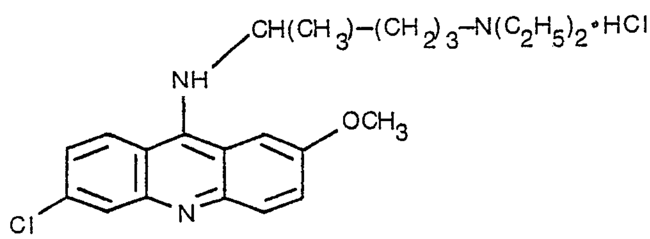

The structure of emetine is

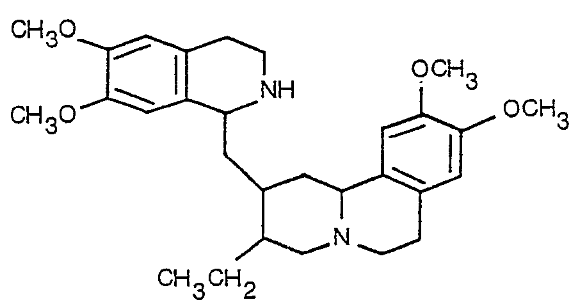

The structure of procaine is

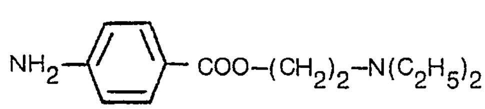

*Approach to Present Invention*

The approach to the present invention has been in part through the inventor's concern with zinc and levamisole among other materials, shown in earlier work of his to be significant to PG metabolism. This work is discussed for example in European Patent Applications Nos. 79300079.5 and

79300546.3 (Publication Nos. 0003407 and 0004770).

Zinc and levamisole under certain conditions are anti-rheumatic agents. Chloroquine also has anti-rheumatic actions, although its mode of action is not at all understood, and it was thus decided to investigate it in the present context. Others of the compounds followed in that chloroquine is closely related chemically and in its clinical uses against protozoal parasites to other 4-aminoquinolines and to the 8-hydroxy and 8-aminoquinolines including diiodohydroxyquin, and shares biological properties (described later herein in relation to noradrenaline responses) with quinacine, quinine, quinidine and procaine, and antiprotozoal properties with emetine. Antiprotozoal action is an unusual pharmaceutical property and while the inventor has no detailed explanation of the property in terms of PG metabolism, when some antiprotozoals have been shown to show favourable action on PG metabolism others will show it also. Their characteristics are discussed for example in Goodman and Gilman, 5th Edition 1975 Ed. E.N. Rollo pp. 1045—1089.

*Experimental Results*

In the case of chloroquine the effect of enhancement of 1-series PG production is demonstrated in results in human platelets and rat kidney slices given below. Other 4-aminoquinolines have the same effect, together with the 8-hydroxy and 8-aminoquinolines which, although their solubility properties may give some difficulty, share both chemical similarity and the related and unusual pharmaceutical properties referred to.

In the work, the detailed procedure of which is given later, human platelets and rat kidney slices were incubated with 14C-labelled arachidonic acid (AA) and dihomo-$\gamma$-linolenic acid (DGLA) in the presence or absence of 5 $\mu$g/ml chloroquine or spironolactone. The conversions of the precursors to the respective PGEs and TXBs in platelets and to the PGEs in kidney slices were investigated. The results are shown in the following tables and are expressed as percentages of the results obtained during control incubations when drug vehicle alone was added. A 10% variation is a reliable indication of real change.

TABLE 1

Kidney slices (rat)

| Precursor acid | DGLA | AA |
|---|---|---|
| Product | PGE 1 | PGE 2 |
| 5 $\mu$g/ml chloroquine | 129% | 101% |

TABLE 2

Platelets (human)

| Precursor acid | DGLA | DGLA | AA | AA |
|---|---|---|---|---|
| Product | PGE 1 | TXB 1 | PGE 2 | TXB 2 |
| 5 $\mu$g/ml chloroquine | 132% | 128% | 98% | 97% |

It is concluded that chloroquine selectively enhances conversion of labelled DGLA to its products while leaving conversion of labelled AA unchanged within the accuracy of measurement. The compound therefore influences the balance of 1-series and 2-series and 2-series PGs in favour of 1-series PGs.

The effect of chloroquine sheds light on another action of chloroquine which has been noted previously (Prostaglandins 12: 789, 1976). Chloroquine inhibits responses to noradrenaline in the rat mesenteric vascular bed. This inhibitory action is more potent when endogenous prostaglandin synthesis is intact than when PG synthesis is blocked by indomethacin or by aspirin. At the time it was not possible to explain this and no explanation has been forthcoming until now. However DGLA and PGE 1 are able to inhibit responses to noradrenaline whereas AA and 2-series PGs in this preparation increase noradrenaline responses. If chloroquine causes a rise in 1-series PGs relative to 2-series PGs, this would explain why it blocks noradrenaline responses more effectively when PG production is intact than when production of both PG series is blocked. We therefore conclude that agents which, like chloroquine, inhibit noradrenaline responses more effectively in the presence of intact PG synthesis, are

likely to be selective enhancers of formation of 1-series PGs. Four compounds tested at that time share this effect of chloroquine. They are quinacrine, quinine and its d-isomer quinidine, and procaine.

*Materials and methods*

The detailed technique with platelets is given below by way of example. That with kidney slices was essentially similar.

(1-$^{14}$C) arachidonic acid and (1-$^{14}$C) dihomo-$\gamma$-linolenic acid was used, diluted with hexane to specific activities of about 2 or 5 $\mu$Ci/$\mu$mol. One day expired (2 days old) human platelets were obtained and used within 48 hours of expiration. One unit was centrifuged at 1000 g for 15 minutes and the supernatant drawn off. The platelet pellet was resuspended in Tris-NaCl-EDTA buffer, made up of 0.15 M NaCl, 0.15 M Tris HCl at pH 7.4 and 0.077 M NaEDTA (90:8:2 v/v/v). The platelets were recentrifuged, the supernatant removed and the pellet resuspended in Krebs-Henseleit buffer (without calcium) at oH 7.4. The washed platelet suspension contained about 1—2% red blood cells. All glassware used in the preparation of the platelets was siliconized.

Four equal sized 1 ml aliquots of the platelet suspension, containing $10^9$ platelets/ml were incubated with e.g. 0.5 $\mu$Ci $^{14}$C—DGLA for five minutes. At the beginning of the incubation the drug under test was added to the suspensions. The reaction was stopped after five minutes by addition of 1/10 volume of 10% formic acid. The suspension was then extracted three times with ethyl acetate and the fractions pooled and dried under vacuum. The extract was then taken up with 5 ml chloroform/methanol (2/1, v/v). Recovery of radioactive material in the extract was checked by taking 50 $\mu$l of the chloroform/methanol and counting by liquid scintillation. Recovery was in the range 80—95% in most experiments.

The chloroform/methanol extract was then reduced in volume to 1 ml under dry prepurified nitrogen. Thin layer chromatography was carried out on 500 $\mu$g precoated, prescored silica gel G Uniplates (Analtech). Plates were activated by heating to 100°C for 1 hour immediately prior to use. The solvent system was chloroform:methanol:acetic acid:water (90:8:1:0.8). Reference compounds (PG's E1 and F1$\alpha$, and thromboxane B1) were run at the same time and visualized by phosphomolybdic acid spray followed by brief heating. The bands on the plates corresponding to the reference PGE 1, PFG 1$\alpha$ and TXB 1 were scraped off and eluted with 20 ml acetone. Each elution was then evaporated to dryness and counted by liquid scintillation (Beckman 100 LS counter).

Using the same bath of platelets at the same time exactly similar experiments were carried out with $^{14}$-C—AA and PGE 2, PGE 2$\alpha$ and TXB 2 as reference compounds. Three experiments were performed with DGLA and three with AA.

*Relationship to previous proposals*

The above approaches may be used in combination with other materials as disclosed in the earlier patent applications referred to above, to which reference may be made.

Among these materials are a number believed to act by enhancing mobilisation of DGLA reserves, including zinc, penicillin and $\beta$-lactam antibiotics generally, and also penicillamine, phenformin and levamisole when the other effects of these materials are acceptable.

Further, since there is evidence that thromboxane A2 may indirectly enhance formation of PGE 1, substances such as colchicine, amantadine and melatonin, as discussed in the pending patent applications and believed to act through increasing the production or effect of thromboxane A2, can also be used in conjunction with the materials of the present invention.

A further group of materials with which those of the present application may be used is disclosed in European Patent Application No. 80301510.6 (Publication No. 0019423) namely Vitamin C (ascorbic acid); ethyl alcohol; and naloxone, nalorphine, levallorphan and other opiate antagonists. These materials are discussed at length in that application, to which reference may be made and are a class that enhance physiological synthesis of 1-series PGs from DGLA without substantially enhancing synthesis of 2-series PGs from AA.

As appears from the earlier patent applications, in searching for ways to regulate PGE 1 formation the inventor has previously concentrated on the conversion of DGLA stores to free DGLA since this is believed to be a key rate-limiting step and sincde it has also been believed that factors which regulate conversion of free arachidonate to PGs will also regulate conversion of free DGLA to PGs. The present work has been more on the conversion of DGLA and of AA to the respective PGs, and as noted above it has been found that the factors regulating the two PG pathways are in some respects quite different. The discoveries on which the present application is based however build on and add to the earlier inventions, rather than superseding them.

*Detailed statement of uses of the present invention*

In the light of the general discussion above the uses of the present invention in its various aspects may be summarised as below. The invention as claimed lies in the compositions referred to, as such, and particularly when for such uses.

A. A method of treating any condition in which enhancement of 1-series PG production, or more broadly influence of the 1-series/2-series balance in the body in favour of 1-series PGs, is indicated, and

particularly, any of the conditions listed earlier herein, which comprises administering an effective amount of (a) γ-linolenic acid and/or dihomo-γ-linolenic acid, optionally in association with linoleic and if desired other fat acids, said acids being used if desired as physiologically functional derivatives thereof, in conjunction with (b) an effective amount of one or more of the agents specified herein.

B. A method as A, comprising further administering an effective amount of (c) a material enhancing physiological synthesis of 1-series PGs, and in particular PGE 1, specifically by mobilisation of DGLA reserves, or in particular of (d) zinc, a β-lactam antibiotic as described herein, penicillamine, levamisole or phenformin.

C. A method as A or B comprising further administering an effective amount of (e) a material enhancing the production or effect of thromboxane A2 in the body or in particular (f) melatonin or colchicine or amantidine or interferon or griseofulvin or vinblastine, vincristine or other Vinca alkaloid.

D. A method as A, B or C comprising further administering an effective amount of (g) ascorbic acid, or ethyl alcohol, or naloxone, nalorphine, levallorphan or other opiate antagonist.

*Dose ranges (materials of present invention)*

The following are effective:

Chloroquine 250 mg/week to 2500 mg/day

(Amounts of amodiaquine and hydroxychloroquine and the 4-amino and 8-amino and 8-hydroxy quinolines named and generally are comparable)

| | |
|---|---|
| Quinacrine<br>(Amounts of other acridines are comparable) | 100 mg to 2500 mg/day |
| Quinine | 100 mg to 10 g/day |
| Quinidine | 100 mg to 2 g/day |
| Procaine | 100 mg to 10 g/day |
| Emetine | 10 mg to 100 mg/day |
| Metronidazole | 100 mg to 10 g/day |

*Packs*

If it is not desired to have compositions comprising the active materials together, as listed above, packs may be prepared comprising the materials presented for separate or part joint and part separate administration in the appropriate relative amounts, and such packs are within the purview of the invention.

*Dietary compositions*

The invention is chiefly described in terms of pharmaceutical compositions, but it will be understood that the γ-linolenic and other acids, being in the nature of dietary supplements, could be incorporated in a dietary margarine or other foodstuffs; such foodstuffs, possibly containing other active materials and generally referred to in this description as dietary or pharmaceutical compositions, are within the purview of the invention and thus of the term pharmaceutical compositions, packs or the like used in the statement of invention and claims.

*Veterinary applications*

It will be understood that where a disorder of a kind calling for treatment in animals arises, the invention while described primarily in terms of human medicine and treatment is equally applicable in the veterinary field.

*Amounts of active materials (adjuncts to present invention)*

Amounts of materials are:

| | | |
|---|---|---|
| I | Zinc | 2.5 to 800 mg/day, preferably 10—80 mg, calculated as zinc |
| | β-lactam antibiotics | 0.5 to 10 g/day |
| | Penicillamine | 50 mg to 10 g/day |
| | Phenformin | 10 mg to 5 g/day |

| | | |
|---|---|---|
| | Levamisole | 10 mg to 2 g/day |
| II | Colchicine | 0.3 to 15 mg/day, preferably 0.6 to 2.4 mg |
| | Melatonin | 10 mg to 5 g/day |
| | Amantadine | 100 mg to 1000 mg/day |
| | Griseofulvin | 0.5 g to 5 g/day |
| | Vinblastine | 35 mg to 350 mg/week |
| | Vincristine | 7 mg to 70 mg/week |
| | Interferon | $1 \times 10^5$ to $1 \times 10^8$ units/day |
| | Melatonin | 10 mg to 5 g/day |
| III | Ascorbic acid | 50 mg to 50 g preferably 250 mg to 5 g/day |
| | Ethyl alcohol | 5 ml to 500 ml preferably 50 ml to 200 ml/day |
| | Naloxone | 0.1 mg to 500 mg preferably 10 mg to 200 mg/day |
| | Nalorphine | 1 mg to 5 g preferably 10 mg to 2 g/day |
| | Levallorphan | 0.2 mg to 1 g preferably 10 mg to 1 g/day |

and comparable amounts of other opiate antagonists.

Detailed discussion of suitable amounts and forms of use is contained in the published patent applications referred to earlier to which reference may be made. In particular the $\beta$-lactam antibiotics are conveniently any of the known penicillin and cephalosporin antibiotics (including semi-synthetic antibiotics) such as, for example, penicillin G, penicillin N, penicillin V, cephalexin, cephalothin, ampicillin, amoxycillin, cloxacillin and cephaloglycin. Any of these may be used in the form of their physiologically functional non-toxic derivatives, for example alkali metal salts e.g. sodium and potassium salts, and salts with organic bases, and reference to an antibiotic herein includes reference to such derivatives.

*Amounts of $\gamma$-linolenic and other acids specifically*

A preferred daily dosage for all purposes for an adult (weight ca 75 kg) is from 0.05 to 0.1 up to 1, 2, 5 or even 10 g as required of $\gamma$-linolenic acid or equivalent weight calculated as $\gamma$-linolenic acid or a physiologically functional derivative thereof. Amounts in particular may be 0.1 to 1.0 g daily. Such doses correspond to about 2 to 20 g daily of the Oenothera oil discussed below. In place of, or in addition to, $\gamma$-linolenic acid, one may use dihomo-$\gamma$-linolenic acid or a physiologically functional derivative thereof, in amounts equivalent in molar terms to $\gamma$-linolenic acid and calculated as such. This dosage can for example be taken as a single dose or divided into 2, 3 or 4 subdivisions thereof as convenient.

*Forms and sources of $\gamma$-linolenic and other acids*

Convenient physiologically functional derivatives of $\gamma$-linolenic acid and dihomo-$\gamma$-linolenic acid for use according to the invention for all the purposes described include the $C_1$—$C_4$ alkyl (e.g. methyl esters and the glycerides of the acids.)

If desired, pharmaceutical compositions may be produced for use in the invention by associating natural or synthetic $\gamma$-linolenic acid (or a physiologically functional derivative thereof) and/or dihomo-$\gamma$-linolenic acid (or a physiologically functional derivative thereof), as such, with an acceptable pharmaceutical vehicle. It is at present convenient to incorporate the $\gamma$-linolenic acid into compositions in the form of an available oil having a high $\gamma$-linolenic acid content, hence references to "oil" herein.

At the present time known natural sources of oils having a high $\gamma$-linolenic acid content are few (there are no known natural sources of significant amounts of dihomo-$\gamma$-linolenic acid). One source of oils currently available is the seed of Evening Primrose species such as *Oenothera biennis L.* and *Oenothera lamarckiana,* the oil extract therefrom containing $\gamma$-linolenic acid (about 8%) and linoleic acid (about 72%) in the form of their glycerides together with other glycerides (percentages based on total fatty acids). Another source of $\gamma$-linolenic acid is Borage species such as *Borago officinalis* which, though its current yield per acre is low, provides a richer source of $\gamma$-linolenic acid than Oenothera oil. Recent studies on fungi which can be cultivated by fermentation promise a fungal oil source.

The seed oil extracts referred to above can be used as such or can for example if desired be fractionated to yield an oily composition containing the triglycerides of $\gamma$-linolenic and linoleic as the main fatty acid components, the $\gamma$-linolenic acid content being if desired a major proportion. Seed oil extracts appear to have a stabilising effect upon any dihomo-$\gamma$-linolenic acid or physiologically functional derivative thereof.

*Pharmaceutical presentation*

The compositions according to the invention are conveniently in a form suitable for oral, rectal, parenteral or topical administration in a suitable pharmaceutical vehicle, as discussed in detail for example in U.K. Patent Specification No. 1 082 624, to which reference may be made, and in any case very well known generally for any particular kind of preparation. Thus for example tablets, capsules, ingestible liquid or powder preparations, creams or lotions for topical application, or suppositories, can be prepared as required. Injectable solutions of hydrolysed Oenothera oil may be prepared using albumin to solubilise the free acid.

Advantageously a preservative is incorporated into the preparations. $\alpha$-Tocopherol in a concentration of about 0.1% by weight has been found suitable for the purpose.

It will be understood that the absolute quantity of active ingredients present in any dosage unit should not exceed that appropriate to the rate and manner of administration to be employed but on the other hand should also desirably be adequate to allow the desired rate of administration to be achieved by a small number of doses. The rate of administration will moreover depend on the precise pharmacological action desired.

The following Examples serve to illustrate pharmaceutical compositions useful in treatment according to the invention.

Examples

Pharmaceutical compositions contain a unit dose of an oil extract from the seeds of *Oenothera biennis L.* and of one of the active materials of the present invention, optionally with methyl dihomo-$\gamma$-linolenate and/or zinc oleate and/or penicillin V or other active materials. They may be presented by encapsulation of the natural oil in soft gelation capsules by known methods.

The oil is extracted from the seeds by one of the conventional methods of extraction such as cold pressure, screw pressure after partially cooking the seed, or solvent extraction.

Fractionation of a typical sample of this oil shows a yield of 97.0% oil in the form of methyl esters, with the relative proportions:

| | |
|---|---|
| Palmitate | 6.15 |
| Stearate | 1.6 |
| Oleate | 10.15 |
| Linoleate | 72.6 |
| $\gamma$-Linolenate | 8.9 |

As preservative, $\alpha$-tocopherol is added to the oil in a concentration of 0.1%.

Gelatin capsules containing oil extracts prepared as described above, each having the following contents of active ingredients (0.5 g oil extract — ca 0.045 g $\gamma$-linolenic acid), are prepared in conventional fashion.

The following are specific examples of capsules that may be given, two capsules three times a day, in treatment of the conditions listed earlier.

Example 1

Capsules containing:

| | |
|---|---|
| Oil extract | 0.5 g |
| Chloroquine | 50 mg |

Alternatives to chloroquine in the same amount are amodiaquine and hydroxychloroquine, and diidohydroxyquin, iodochlorhydroxyquin, chiniofon, pentaquine, isopentaquine and primaquine.

Example 2

Capsules containing:

| | |
|---|---|
| Oil extract | 0.5 g |
| Quinacrine | 50 mg |

Alternatives to chloroquine in the same amount are amodiaquine and hydroxychloroquine, and diidohydroxyquin, iodochlorhydroxyquin, chiniofon, pentaquine, isopentaquine and primaquine.

Example 2

Capsules containing:

| | |
|---|---|
| Oil extract | 0.5 g |
| Quinacrine | 50 mg |

Example 3

Capsules containing:

| | |
|---|---|
| Oil extract | 0.5 g |
| Quinine or quinidine | 50 mg |

Example 4

Capsules containing:

| | |
|---|---|
| Oil extract | 0.5 g |
| Emetine | 50 mg |
| or Metronidazole | 200 mg |

Example 5

Capsules containing:

| | |
|---|---|
| Oil extract | 0.5 g |
| Procaine | 100 mg |

Similarly capsules containing additional materials may be administered, for example, 10 mg methyl dihomo-$\gamma$-linolenate per capsule as a direct supplement to the oil; or for example for their indirect action zinc oleate 10 mg or penicillin V 0.25 g (compare the Examples of European Patent Application No. 79300079.5 or U.S. Patent Application No. 004 924 referred to earlier); or phenformin 25 mg, levamisole 25 mg or penicillamine 100 mg per capsule, or colchicine 0.2 mg, amantadine 100 mg or griseofulvin 0.5 mg per capsule (compare the Examples of European Patent Application No. 79300546.3 referred to earlier and also the use in conjunction with vincristine, vinblastine, melatonin and interferon referred to therein); or ascorbic acid 100 mg, naloxone 5 mg, nalorphine 5 mg or levallorphan 5 mg (compare the Examples of European Patent Application No. 80301510.6 or U.S. Patent Application No. 150 402 referred to earlier and also the use in conjunction with ethyl alcohol to give 30 to 300 mg% alcohol in the body referred to therein).

It will be understood throughout that while a full theoretical discussion of what is believed to be the reason for the effectiveness of the compositions proposed is given to aid understanding, the scope of the claims is in no way to be limited by this discussion.

**Claims for the Contracting States: BE CH DE FR GB IT LI LU NL SE**

1. A pharmaceutical composition comprising $\gamma$-linolenic acid and/or dihomo-$\gamma$-linolenic acid, or a salt, ester or other derivative thereof convertible in the body thereto, together with a compound influencing the 1-series/2-series PG balance in the body in favour of 1-series PGs and selected from chloroquine and other 4-aminoquinolines including amodiaquine and hydroxychloroquine; diiodohydroxyquin and other 8-hydroxy and 8-aminoquinolines including iodochlorhydroxyquin,

chiniofon, pentaquine, isopentaquine and primaquine; quinacrine (mepacrine) and other acridines; quinidine, quinine and procaine; and emetine and metronidazole alone or in an acceptable pharmaceutical vehicle.

2. A composition according to claim 1, presented for administration in quantities to give doses of 250 mg/week to 2500 mg/day of said quinoline derivative; 100 to 2500 mg/day of said acridine derivative; 100 mg to 10 g/day of quinine, 100 mg to 2 g/day of quinidine, or 100 mg to 10 g/day of procaine; 30 mg to 2 g/day of said steroid; 10 to 100 mg/day of emetine or 100 mg to 10 g/day of metronidazole.

3. A composition according to claim 1, presented for administration in quantities to give 0.05 to 10 g/day of said $\gamma$-linolenic or dihomo-$\gamma$-linolenic acid or derivative, calculated as $\gamma$-linolenic acid.

4. A composition according to claim 1, further comprising physiologically assimible zinc, a $\beta$-lactam antibiotic, penicillamine, phenformin or levamisole.

5. A composition according to claim 4, presented for administration in quantities to give 2.5 to 800 mg/day zinc, 0.5 to 10 g/day $\beta$-lactam antibiotic, 50 mg to 10 g/day penicillamine, 10 mg to 5 g/day phenformin or 10 mg to 2 g/day levamisole.

6. A composition according to claim 1 further comprising a material selected from colchicine, griseofulvin, amantadine, melatonin, interferon, and vinblastine, vincristine and other Vinca alkaloids.

7. A composition according to claim 6, presented for administration in quantities to give doses of:
0.3 to 15 mg/day colchicine
100 to 1000 mg/day amantadine
0.5 to 5 g/day griseofulvin
35 to 350 mg/week vinblastine
7 to 70 mg/week vincristine
$1 \times 10^5$ to $1 \times 10^8$ units/day interferon, or
10 mg to 5 g/day melatonin.

8. A composition according to claim 1, further comprising a material selected from Vitamin C, ethyl alcohol, and naloxone, nalorphine, levallorphan and other opiate antagonists.

9. A composition according to claim 8, presented for administration in quantities to give doses of:
50 mg to 50 g/day ascorbic acid or
5 to 500 ml/day ethyl alcohol or
0.1 to 500 mg/day naloxone, 1 mg to 5 g/day nalorphine, 0.2 mg to 1 g/day levallorphan or comparable amount of other opiate antagonist

10. A composition according to any preceding claim, wherein the $\gamma$-linolenic acid is present in the form of the oil of the seed of Oenothera biennis, O. lamarckia or other Evening Primrose species, or a fraction thereof.

11. A composition according to any preceding claim, wherein the $\gamma$-linolenic acid is present in the form of the oil of the seed of Oenothera biennis, O. lamarckiana or other Evening Primrose species, or a

12. A composition according to any preceding claim, when for use is therapy for influencing the 1-series/2-series PG balance in the body in favour of 1-series PG's.

13. A pharmaceutical pack comprising the materials set out in any preceding claim presented separately, or one or more separately and others together, but for conjoint administration.

**Claims for the Contracting State: AT**

1. The use of $\gamma$-linolenic acid and/or dihomo-$\gamma$-linolenic acid, or a salt, ester or other derivative thereof convertible in the body thereto, together with a compound influencing the 1-series/2-series PG balance in the body in favour of 1-series PGs and selected from chloroquine and other 4-aminoquinolines including amodiaquine and hydroxychloroquine; diiodohydroxyquin and other 8-hydroxy and 8-aminoquinolines including iodochlorhydroxyquin, chiniofon, pentaquine, isopentaquine and primaquine; quinacrine (mepacrine) and other acridines; quinidine, quinine and procaine; and emetine and metronidazole alone or in an acceptable pharmaceutical vehicle for the method of producing a pharmaceutical composition for influencing the 1-series/2-series PG balance in the body in favour of 1-series PG's.

2. The use of claim 1, the composition being presented for administration in quantities to give doses of 250 mg/week to 2500 mg/day of said quinoline derivative; 100 to 2500 mg/day of said acridine derivative; 100 mg to 10 g/day of quinine, 100 mg to 2 g/day of quinidine, or 100 mg to 10 g/day of procaine; 30 mg to 2 g/day of said steroid; 10 to 100 mg/day of emetine or 100 mg to 10 g/day of metronidazole.

3. The use of claim 1, the composition being presented for administration in quantities to give 0.05 to 10 g/day of said $\gamma$-linolenic or dihomo-$\gamma$-linolenic acid or derivative, calculated as $\gamma$-linolenic acid.

4. The use of claim 1, the composition being further comprising physiologically assimible zinc, a $\beta$-lactam antibiotic, penicillamine, phenformin or levamisole.

5. The use of claim 4, the composition being presented for administration in quantities to give 2.5 to 800 mg/day zinc, 0.5 to 10 g/day $\beta$-lactam antibiotic, 50 mg to 10 g/day penicillamine, 10 mg to 5

g/day phenformin or 10 mg to 2 g/day levamisole.

6. The use of claim 1, the composition being further comprising a material selected from colchicine, griseofulvin, amantadine, melatonin, interferon, and vinblastine, vincristine and other Vinca alkaloids.

7. The use of claim 6, the composition being presented for administration in quantities to give doses of:

0.3 to 15 mg/day colchicine
100 to 1000 mg/day amantadine
0.5 to 5 g/day griseofulvin
35 to 350 mg/week vinblastine
7 to 70 mg/week vincristine
$1 \times 10^5$ to $1 \times 10^8$ units/day interferon, or
10 mg to 5 g/day melatonin.

8. The use of claim 1, the composition being further comprising a material selected from Vitamin C, ethyl alcohol, and naloxone, nalorphine, levallorphan and other opiate antagonists.

9. The use of claim 8, the composition being presented for administration in quantities to give doses of:

50 mg to 50 g/day ascorbic acid or
5 to 500 ml/day ethyl alcohol or
0.1 to 500 mg/day naloxone, 1 mg to 5 g/day nalorphine, 0.2 mg to 1 g/day levallorphan or comparable amount of other opiate antagonist.

10. The use of any preceding claim, wherein the $\gamma$-linolenic acid is present in the form of the oil of the seed of Oenothera biennis, O. lamarckiana or other Evening Primrose species, or a fraction thereof.

11. The use of any preceding claim, wherein the $\gamma$-linolenic acid is present in the form of the oil of the seed of Borago officinalis or other Borage species or a fraction thereof.

**Patentansprüche für die Vertragsstaaten: BE CH DE FR GB IT LI LU NL SE**

1. Pharmazeutisches Präparat, bestehend aus $\gamma$-Linolensäure und/oder Dihomo-$\gamma$-Linolensäure oder deren im Körper dazu umwandelbarem Salz, Ester oder sonstigem Derivat und einer Verbindung, die das Gleichgewicht von 1.Serie-Prostaglandin zu 2.Serie-Prostaglandin im Körper zugunsten der 1.Serie-Prostaglandine beeinflußt, ausgewählt aus: Chlorochin und anderen 4-Aminochinolinen, einschließlich Amodiachin und Hydroxychlorochin; Dijohydroxychin und anderen 8-Hydroxy- und 8-Aminochinolinen, einschließlich Jodchlorhydroxychin, Chiniofon, Pentachin, Isopentachin und Primachin; Chinakrin (Mepacrin) und anderen Akridinen; Chinidin, Chinin und Procain; sowie Emetin und Metronidazol, jeweils für sich oder in einem verträglichen Arzneistoffträger.

2. Präparat nach Anspruch 1, dargeboten zur Verabreichung in solchen Mengen, daß sich Dosen zwischen 250 mg/Woche und 2500 mg/Tag des Chinolin-Derivats, zwischen 100 und 2500 mg/Tag des Akridin-Derivats, zwischen 100 mg und 10 g/Tag Chinin, zwischen 100 mg und 2 g/Tag Chinidin oder zwischen 100 mg und 10 g/Tag Procain, ferner zwischen 30 mg und 2 g/Tag des Steroids sowie zwischen 10 und 100 mg/Tag Emetin oder zwischen 100 mg und 10 g/Tag Metronidazol ergeben.

3. Präparat nach Anspruch 1, dargeboten zur Verabreichung in solchen Mengen, daß sich Dosen zwischen 0,05 und 10 g/Tag $\gamma$-Linolensäure oder Dihomo-$\gamma$-Linolensäure oder -Derivate ergeben, gerechnet als $\gamma$-Linolensäure.

4. Präparat nach Anspruch 1, enthaltend außerdem physiologisch assimilierbares Zink, ein $\beta$-Laktam-Antibiotikum, Penicillamin, Phenformin und Levamisol.

5. Präparat nach Anspruch 4, dargeboten zur Verabreichung in solchen Mengen, daß sich Dosen zwischen 2,5 und 800 mg/Tag Zink, zwischen 0,5 und 10 g/Tag $\beta$-Laktam-Antibiotikum, zwischen 50 mg und 10 g/Tag Penicillamin, zwischen 10 mg und 5 g/Tag Phenformin oder zwischen 10 mg und 2 g/Tag Levamisol ergeben.

6. Präparat nach Anspruch 1, außerdem enthaltend eine der folgenden Substanzen: Colchicin, Griseofulvin, Amantadin, Melatonin, Interferon und Vinblastin, Vincristin und andere Vinca-Alkaloide.

7. Präparat nach Anspruch 6, dargeboten zur Verabreichung in solchen Mengen, daß sich folgende Dosen ergeben:

0,3 bis 15 mg/Tag Colchicin
100 bis 1000 mg/Tag Amantadin
0,5 bis 5 g/Tag Griseofulvin
35 bis 350 mg/Woche Vinblastin
7 bis 70 mg/Woche Vincristin
$1 \times 10^5$ bis $1 \times 10^8$ Einheiten/Tag Interferon oder
10 mg bis 5 g/Tag Melatonin.

8. Präparat nach Anspruch 1, außerdem enthaltend eine der folgenden Substanzen: Vitamin C, Ethylalkohol und Naloxon, Nalorphin, Levallorphan und andere Opiat-Antagonisten.

9. Präparat nach Anspruch 8, dargeboten zur Verabreichung in solchen Mengen, daß sich Dosen zwischen 50 mg und 50 g/Tag Ascorbinsäure oder

zwischen 5 und 500 ml/Tag Ethylalkohol oder
zwischen 0,1 und 500 mg/Tag Naloxon, 1 mg und 5 g/Tag Nalorphin, 0,2 mg und 1 g/Tag Levallorphan oder einer vergleichbaren Menge anderer Opiat-Antagonisten
ergeben.

10. Präparat nach einem der vorhergehenden Ansprüche, dadurch gekennzeichnet, daß die γ-Linolensäure in Form des Öls der Samen von Oenothera biennis L., Oenothera lamarckiana oder einer anderen Nachtkerzenart oder einer Fraktion derselben vorliegt.

11. Präparat nach einem der vorhergehenden Ansprüche, dadurch gekennzeichnet, daß die γ-Linolensäure in Form des Öls der Samen von Borago officinals oder einer anderen Boragoart oder einer Fraktion derselben vorliegt.

12. Präparat nach einem der vorhergehenden Ansprüche, angewandt in der Therapie zur Beeinflussung des Gleichgewichts von 1.Serie-Prostaglandin zu 2.Serie-Prostaglandin zugunsten der 1.Serie-Prostaglandine.

13. Pharmazeutische Packung, bestehend aus den in einem der vorhergehenden Ansprüche genannten Substanzen, dargeboten getrennt oder mindestens eine Substanz getrennt und andere zusammen, aber zur gemeinsamen Verabreichung bestimmt.

**Patentansprüche für den Vertragsstaat: AT**

1. Verwendung von γ-Linolensäure und/oder Dihomo-γ-Linolensäure oder deren im Körper dazu umwandelbarem Salz, Ester oder sonstigem Derivat, und einer Verbindung, die das Gleichgewicht von 1.Serie-Prostaglandin zu 2.Serie-Prostaglandin im Körper zugunsten der 1.Serie-Prostaglandine beeinflußt, ausgewählt aus: Chlorochin und anderen 4-Aminochinolinen, einschließlich Amodiachin und Hydroxychlorochin; Dijodhydroxychin und anderen 8-Hydroxy- und 8-Aminochinolinen, einschließlich Jodchlorhydroxychin, Chiniofon, Pentachin, Isopentachin und Primachin; Chinakrin (Mepacrin) und anderen Akridinen; Chinidin, Chinin und Procain; sowie Emetin und Metronidazol, jeweils für sich oder in einem verträglichen Arzneistoffträger, für das Verfahren der Herstellung eines pharmazeutischen Präparats zur Beeinflussung des Gleichgewichts von 1.Serie-Prostaglandin zu 2.Serie Prostaglandin im Körper zugunsten der 1.Serie-Prostaglandine.

2. Verwendung nach Anspruch 1, dadurch gekennzeichnet, daß das Präparat zur Verabreichnung in solchen Mengen dargeboten wird, daß sich Dosen zwischen 250 mg/Woche und 2500 mg/Tag des Chinolin-Derivats, zwischen 100 und 2500 mg/Tag des Akridin-Derivats, zwischen 100 mg und 10 g/Tag Chinin, zwischen 100 mg und 2 g/Tag Chinidin oder zwischen 100 mg und 10 g/Tag Procain, ferner zwischen 30 mg und 2 g/Tag des Steroids sowie zwischen 10 und 100 mg/Tag Emetin oder zwischen 100 mg und 10 g/Tag Metronidazol ergeben.

3. Verwendung nach Anspruch 1, dadurch gekennzeichnet, daß das Präparat zur Verabreichung in solchen Mengen dargeboten wird, daß sich Dosen zwischen 0,05 und 10 g/Tag γ-Linolensäure oder Dihomo-γ-Linolensäure oder -Derivate ergeben, gerechnet als γ-Linolensäure.

4. Verwendung nach Anspruch 1, dadurch gekennzeichnet, daß das Präparat außerdem physiologisch assimilierbares Zink, β-Laktam-Antibiotikum, Penicillamin, Phenformin oder Levamisol enthält.

5. Verwendung nach Anspruch 1, dadurch gekennzeichnet, daß das Präparat zur Verabreichnung in solchen Mengen dargeboten wird, daß sich Dosen zwischen 2,5 und 800 mg/Tag Zink, zwischen 0,5 und 10 g/Tag β-Laktam-Antibiotikum, zwischen 50 mg und 10 g/Tag Penicillamin, zwischen 10 mg und 5 g/Tag Phenformin oder zwischen 10 mg und 2 g/Tag Levamisol ergeben.

6. Verwendung nach Anspruch 1, dadurch gekennzeichnet, daß das Präparat außerdem eine der folgenden Substanzen enthält: Colchicin, Griseofulvin, Amantadin, Melatonin, Interferon und Vinblastin, Vincristin und andere Vinca-Alkaloide.

7. Verwendung nach Anspruch 6, dadurch gekennzeichnet, daß das Präparat zur Verabreichnung in solchen Mengen dargeboten wird, daß sich folgende Dosen ergeben:
0,3 bis 15 mg/Tag Colchicin
100 bis 1000 mg/Tag Amantadin
0,5 bis 5 g/Tag Griseofulvin
35 bis 350 mg/Woche Vinblastin
7 bis 70 mg/Woche Vincristin
1 × $10^5$ bis 1 × $10^8$ Einheiten/Tag Interferon oder
10 mg bis 5 g/Tag Melatonin.

8. Verwendung nach Anspruch 1, dadurch gekennzeichnet, daß das Präparat außerdem eine der folgenden Substanzen enthält: Vitamin C, Ethylalkohol und Naloxon, Nalorphin, Levallorphan und andere Opiat-Antagonisten.

9. Verwendung nach Anspruch 8, dadurch gekennzeichnet, daß das Präparat zur Verabreichung in solchen Mengen dargeboten wird, daß sich Dosen
zwischen 50 mg und 50 g/Tag Ascorbinsäure oder
zwischen 5 und 500 ml/Tag Ethylalkohol oder
zwischen 0,1 und 500 mg/Tag Naloxon, 1 mg und 5 g/Tag Nalorphin, 0,2 mg und 1 g/Tag Levallorphan oder einer vergleichbaren Menge anderer Opiat-Antagonisten ergeben.

10. Verwendung nach einem der vorhergehenden Ansprüche, dadurch gekennzeichnet, daß die $\gamma$-Linolensäure in Form des Öls der Samen von Oenothera biennis L., Oenothera lamarckiana oder einer anderen Nachtkerzenart oder einer Fraktion derselben vorliegt.

11. Verwendung nach einem der vorhergehenden Ansprüche, dadurch gekennzeichnet, daß die $\gamma$-Linolensäure in Form des Öls der Samen von Borago officinalis oder einer anderen Boragoart oder einer Fraktion derselben vorliegt.

**Revendications pour les Etats contractants: BE CH DE FR GB IT LI LU NL SE**

1. Une composition pharmaceutique comprenant de l'acide $\gamma$-linolénique et/ou de l'acide dihomo-$\gamma$-linolénique, ou un de leurs sels ou esters ou d'autres dérivés convertibles en ces acides dans l'organisme, avec un composé qui influence l'équilibre PG de la série l/PG de la série 2 dans l'organisme en faveur des PG de la série 1 et choisi parmi la chloroquine et d'autres 4-aminoquinoléines, comprenant l'amodiaquine et l'hydroxychloroquine; la diiodohydroxyquine et d'autres 8-hydroxy et 8-aminoquinoléines comprenant l'iodochlorhydroxyquine, le chiniofon, la pentaquine, l'isopentaquine et la primaquine; la quinacrine (mépacrine) et d'autres acridines; la quinidine, la quinine et la procaïne; et l'émétine et le métronidazole, seuls ou dans un véhicule pharmaceutique acceptable.

2. Une composition selon la revendication 1, présentée pour l'administration en quantités donnant des doses de 250 mg par semaine à 2500 mg par jour dudit dérivé de quinoléine; 100 à 2500 mg par jour dudit dérivé d'acridine; 100 mg à 10 g par jour de quinine, 100 mg à 2 g par jour de quinidine, ou 100 mg à 10 g par jour de procaïne; 30 mg à 2 g par jour dudit stéroïde; 10 à 100 mg par jour d'émétine ou 100 mg à 10 g par jour de métronidazole.

3. Une composition selon la revendication 1 présentée pour l'administration en quantités donnant 0,05 à 10 g/jour dudit acide $\gamma$-linolénique ou dihomo-$\gamma$-linolénique ou son dérivé, calculés en acide $\gamma$-linolénique.

4. Une composition selon la revendication 1, comprenant en outre du zinc physiologiquement acceptable, un antibiotique du type $\beta$-lactame, de la pénicillamine, de la phenformine ou du lévamisole.

5. Une composition selon la revendication 4, présentée pour l'administration en quantités donnant 2,5 à 800 mg/jour de zinc, 0,5 à 10 g/jour d'antibiotique du type $\beta$-lactame, 50 mg à 10 g/jour de pénicillamine, 10 mg à 5 g/jour de phenformine ou 10 mg à 2 g/jour de lévamisole.

6. Une composition selon la revendication 1, comprenant en outre un produit choisi parmi la colchicine, la griséofulvine, l'amantadine, la mélatonine, l'interféron et la vinblastine, la vincristine et d'autres alcaloïdes de Vinca.

7. Une composition selon la revendication 6, présentée pour l'administration en quantités donnant des doses de

0,3 à 15 mg/jour de colchicine
100 à 1000 mg/jour d'amantadine
0,5 à 5 g/jour de griséofulvine
35 à 350 mg/semaine de vinblastine
7 à 70 mg/semaine de vincristine
$1 \times 10^5$ à $1 \times 10^8$ unités/jour d'interféron, ou
10 mg à 5 g/jour de mélatonine.

8. Une composition selon la revendication 1, comprenant en outre un produit choisi parmi la vitamine C, l'alcool éthylique, la naloxone, la nalorphine, le lévallorphan et d'autres antagonistes d'opiacées.

9. Une composition selon la revendication 8, présentée pour l'administration en quantités donnant des doses de

50 mg à 50 g/jour d'acide ascorbique ou
5 à 500 ml/jour d'alcool éthylique ou
0,1 à 500 mg/jour de naloxone,
1 mg à 5 g/jour de nalorphine,
0,2 mg à 1 g/jour de lévallorphan ou une quantité comparable d'un autre antagoniste d'opiacées.

10. Une composition selon l'une quelconque des revendications précédentes, dans laquelle l'acide $\gamma$-linolénique est présent sous la forme de l'huile de graines d'Oenothera biennis, O. lamarckiana ou autres espèces d'oenothère, ou une fraction de cette huile.

11. Une composition selon l'une quelconque des revendications précédentes, dans laquelle l'acide $\gamma$-linolénique présent sous la forme de l'huile de graines de Borago officinalis ou d'une autre espèce de bourrache, ou une fraction de cette huile.

12. Une composition selon l'une quelconque des revendications précédentes, pour l'utilisation dans la thérapie pour influencer l'équilibre PG de la série 1/PG de la série 2 dans l'organisme en faveur des PG de la série 1.

13. Un sachet pharmaceutique comprenant les produits indiqués dans l'une quelconque des revendications précédentes, présentés séparément ou un ou plusieurs séparément et d'autres

ensemble, mais pour l'administration conjointe.

**Revendications pour l'Etat contractant: AT**

1. L'utilisation d'acide $\gamma$-linolénique et/ou d'acide de dihomo-$\gamma$-linolénique, ou un de leurs sels ou esters ou d'autres dérivés convertibles en ces acides dans l'organisme, avec un composé qui influence l'équilibre PG de la série 1 et choisi parmi la chloroquine et d'autres 4-aminoquinoléines, comprenant l'amodiaquine et l'hydroxychloroquine; la diiodohydroxyquine et d'autres 8-hydroxy et 8-aminoquinoléines comprenant l'iodochlorhydroxyquine, le chiniofon, la pentaquine, l'isopentaquine et la primaquine; la quinacrine (mépacrine) et d'autres acridines; la quinidine, la quinine et la procaïne; et l'émétine et le métronidazole seul ou dans un véhicule pharmaceutique acceptable, pour la méthode de production d'une composition pharmaceutique pour influencer l'équilibre PG de la série 1/PG de la série 2 dans l'organisme en faveur des PG de la série 1.

2. L'utilisation de la revendication 1, la composition étant présentée pour l'administration en quantités donnant des doses de 250 mg par semaine à 2500 mg par jour dudit dérivé de quinoléine; 100 à 2500 mg par jour dudit dérivé d'acridine; 100 mg à 10 g par jour de quinne, 100 mg à 2 g par jour de quinidine, ou 100 mg à 10 g par jour de procaïne; 30 mg à 2 g par jour dudit stéroïde; 10 à 100 mg par jour d'émétine ou 100 mg à 10 g par jour de métronidazole.

3. L'utilisation de la revendication 1, la composition étant présentée pour l'administration en quantités de 0,05 à 10 g/jour dudit acide $\gamma$-linolénique ou dihomo-$\gamma$-linolénique ou son dérivé, calculés en acide $\gamma$-linolénique.

4. L'utilisation de la revendication 1, la composition comprenant en outre du zinc physiologiquement acceptable, un antibiotique du type $\beta$-lactame, de la pénicillamine, de la phenformine ou du lévamisole.

5. L'utilisation de la revendication 4, la composition étant présentée pour l'administration en quantités donnant 2,5 à 800 mg/jour de zinc, 0,5 à 10 g/jour d'antibiotique du type $\beta$-lactame, 50 mg à 10 g/jour de pénicillamine, 10 mg à 5 g/jour de phenformine ou 10 mg à 2 g/jour de lévamisole.

6. L'utilisation de la revendication 1, la composition comprenant en outre la colchicine, la griséofulvine, l'amantadine, la mélatonine, l'interféron et la vinblastine, la vincristine et d'autres alcaloïdes Vinca.

7. L'utilisation de la revendication 6, la composition étant présentée pour l'administration en quantités donnant des doses de:

0,3 à 15 mg/jour de colchicine
100 à 1000 mg/jour d'amantadine
0,5 à 5 g/jour de griséofulvine
35 à 350 mg/semaine de vinblastine
7 à 70 mg/semaine de vincristine
$1 \times 10^5$ à $1 \times 10^8$ unités/jour d'interféron, ou
10 mg à 5 g/jour de mélatonine.

8. L'utilisation de la revendication 1, la composition comprenant en outre un produit choisi parmi la vitamine C, l'alcool éthylique, la naloxone, la nalorphine, le lévallorphan et d'autres antagonistes d'opiacées.

9. L'utilisation de la revendication 8, la composition étant présentée pour l'administration en quantités donnant des doses de:

50 mg à 50 g/jour d'acide ascorbique ou
5 à 500 ml/jour d'alcool éthylique ou
0,1 à 500 mg/jour de naloxone,
1 mg à 5 g/jour de nalorphine,
0,2 mg à 1 g/jour de lévallorphan ou une quantité comparable d'un autre antagoniste d'opiacées.

10. L'utilisation de l'une quelconque des revendications précédentes, dans laquelle l'acide $\gamma$-linolénique est présent sous la forme de l'huile de graines d'Oenothera biennis, O. lamarckiana ou autres espèces d'oenothère, ou une fraction de cette huile.

11. L'utilisation de l'une quelconque des revendications précédentes, dans laquelle l'acide $\gamma$-linolénique présent sous la forme de l'huile de graines de Borago officinalis ou d'une autre espèce de bourrache, ou une fraction de cette huile.